# EUROPEAN PATENT APPLICATION

(11) **EP 3 228 284 A1**
(43) Date of publication of application: **11.10.2017**
(21) Application number: 17164634.2
(22) Date of filing: 03.04.2017
(51) Int. Cl.: A61F 5/37, A61G 1/044, A61G 1/04, A41D 13/05, A42B 3/04, A61G 13/12

(54) **SYSTEM FOR IMMOBILIZING THE HEAD AND THE CERVICAL REGION OF A PATIENT**

(30) Priority: 06.04.2016 IT UA20162340
(71) Applicant: Congiu, Giacinto, 08020 Budoni, OT (IT)
(72) Inventor: Congiu, Giacinto, 08020 Budoni, OT (IT)
(74) Representative: Modiano, Micaela Nadia

(57) **Abstract**

A system for immobilizing the head and the cervical region of a patient, which comprises a body (1) that is provided with a collar (2) that is adapted to receive the cervical part and the head of a patient and at least two longitudinal elements (3,3') which are connected to the collar (2) and are configured to engage respectively a right portion and a left portion of the chest of the patient; means (4) of connection to a device for carrying the patient and means (5) of rotation of at least one (3') of the longitudinal elements (3,3') with respect to the collar (2) being provided.

## Description

The present invention relates to a system for immobilizing the head and the cervical region of a patient.

Immobilization devices make it possible to limit the movements of one or more parts of the body of a patient who has been victim of an accident in order to prevent the onset or aggravation of lesions owing to movements by the patient or the patient being carried by first response personnel. Such devices include for example cervical collars, spinal boards, and devices for immobilizing the limbs.

During the first response phase, the presence is required of various qualified personnel who, using suitable techniques, are capable of putting the devices on the patient, who is often unconscious.

Although such conventional devices are appreciable, they are not devoid of drawbacks which include the fact that they are open to human error, given that incorrect maneuvers can have damaging or painful consequences for the patient. Such errors can be minimized by availing of specialist personnel, but a not insignificant percentage of errors is still possible.

Furthermore, conventional devices do not enable an optimal immobilization of the patient, especially during the step of carrying him/her.

A further drawback is due to the fact that the first response personnel have to ensure that the patient is kept in the correct position and wears the device while being carried. Carrying can subject the patient to dangerous stresses, for example owing to the path taken, typically at high speed, by first response vehicles.

Particularly delicate are first response operations that involve the neck and the head of the patient, sites of important organs and nerve endings, which usually require the presence of at least three first response personnel who suitably immobilize various parts of the body of the patient, and move his/her body, for example rotating it, arrange it on a spinal board, affix it thereto with straps and apply a device for immobilizing the head of the patient.

Typically such device for immobilizing the head comprises or is connected to cushions that are arranged so as to rest on the shoulders and on the head of the patient so as to offer resistance to stresses. The application of such head-immobilization device, and in particular of the cushions, is a very delicate and complicated operation. In fact the cushions have to be supported by a paramedic but access must not be impeded to the neck of the patient and the ears must not be obstructed, and any straps that serve to fix the device to the spinal board must be tensioned sufficiently to prevent the patient from moving but not excessively so as to cause pain or injury to the patient, and the operators must make sure the head immobilization device is kept in the correct position during the movement of the patient.

In the known art there are also "HANS" (Head And Neck Support) systems, which are safety supports used in many motor sports and in motocross competitions.

Such conventional HANS systems are worn directly by the driver or rider and their function is to prevent and reduce the risk of injury to the head-neck complex, such as fracture of the base of the skull owing to accidents.

The aim of the present invention is to overcome the above mentioned drawbacks of the known art, by providing a new system that is capable of making the immobilization of the head and the cervical region of a patient easier and safer.

Within this aim, an object of the present invention is to reduce the risk of trauma to the patient as a result of human intervention or of external forces owing to being carried.

Another object of the invention is to reduce the stresses the patient is subjected to and the movements of that patient, so as to limit the pain suffered.

Another object of the invention is to not obstruct the ears, the neck and the head of the patient.

Another object of the present invention is to provide a simple structure which is easy and practical to implement, safe in use and effective in operation, and low cost.

This aim and these and other objects which will become better apparent hereinafter are achieved by the system according to claim 1, a front band according to claim 13 and a spinal board according to claim 14.

Advantageously, the system is modular and allows connection to other components adapted to contribute to immobilizing the patient.

Conveniently, the system makes it possible to safeguard the cervical column of a traumatized patient.

Preferably, the system makes it possible to effectively provide first aid to patients who are victims of road accidents.

Further characteristics and advantages of the invention will become better apparent from the detailed description that follows, which is given in the form of a non-limiting example with accompanying drawings wherein:
Figure 1 is a perspective view of the system according to the invention;
Figure 2 is a front elevation view of the system according to the invention in a first configuration of use;
Figure 3 is a second front elevation view of the system according to the invention in a second configuration of use;
Figure 4 is a detailed rear view of the system according to the invention;
Figure 5 is a view from above of the system according to the invention, connected to a spinal board in the second configuration of use;
Figure 6 is a view from above of the system according to the invention, connected to a spinal board applied to a patient in the first configuration of use.

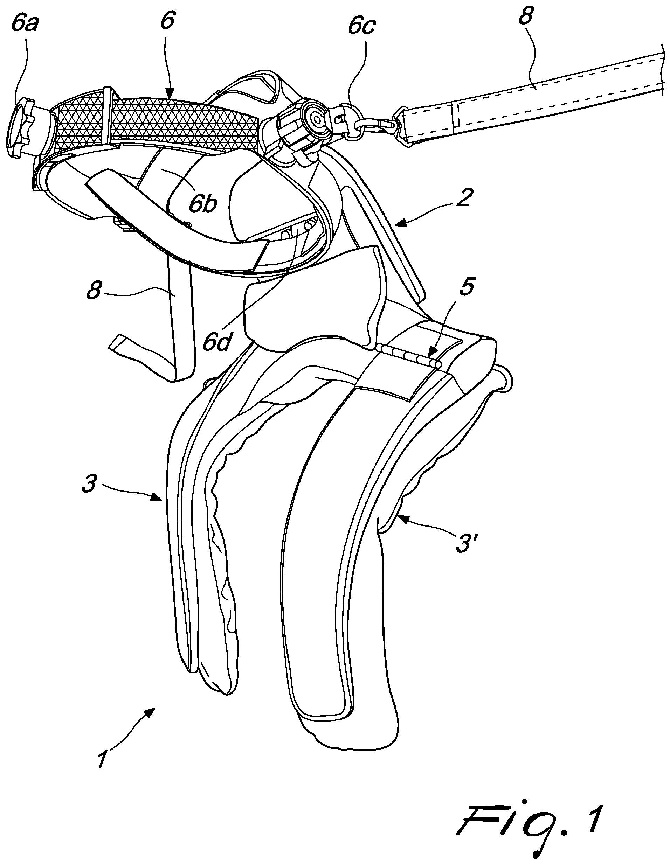

An illustrative architecture of the system according to the present invention is shown in Figure 1.

The system for immobilizing the head and the cervical region of a patient comprises a body 1, which is preferably made of carbon fibers or fiberglass reinforced plastic. The body 1 comprises a collar 2 and two longitudinal elements 3, 3'.

The collar 2 is adapted to receive the cervical part and the head of a patient. The shape structure of the collar is ergonomic and has an adapted seat 2a, preferably approximately 15 cm in diameter, in which it is possible to lay the head of the patient. The collar 2 further has a second seat 2b which is adapted to support the shoulders of the patient, in particular the area corresponding to the trapezius muscle. Preferably the collar 2 has a similar shape to collars of the "HANS" type.

Advantageously, the collar 2 comprises a first padding and a second padding, for example made of sponge rubber, which are arranged respectively in the region corresponding to the seats 2a and 2b which make its use by the patient more comfortable.

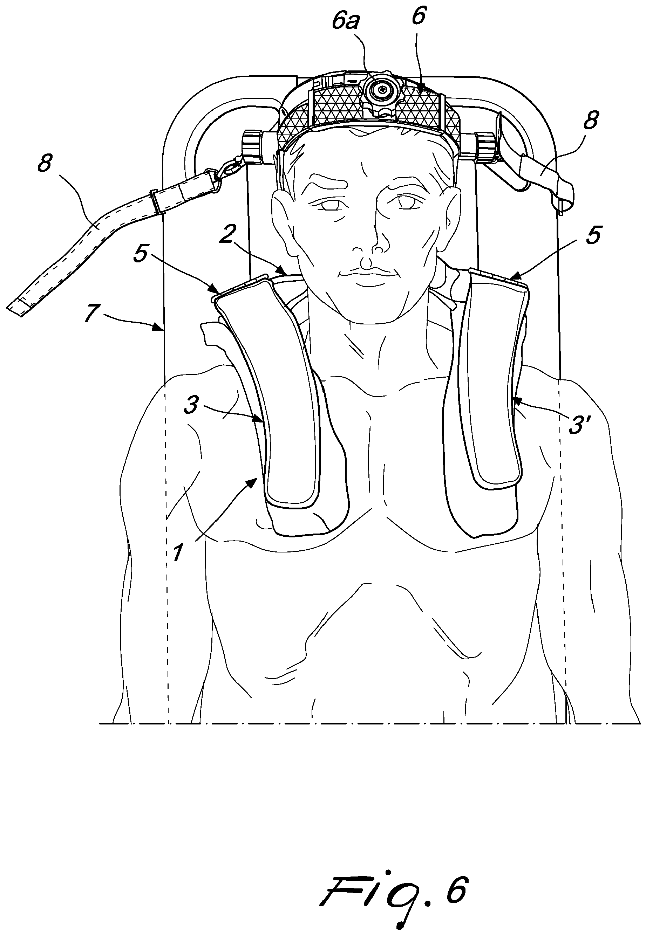

The two longitudinal elements 3, 3' are connected to the collar 2 and are configured to engage respectively a right portion and a left portion of the chest of the patient, as better shown in Figure 6. The longitudinal elements 3, 3' preferably have a contoured curved shape so as to be placed on part of the shoulders and on the chest of the patient, and they have a length preferably of approximately 30 cm. Advantageously, the longitudinal elements 3, 3' pass by the sides of the neck and extend at least until the upper part of the chest.

Preferably, the longitudinal elements 3, 3', are provided with pads of soft material, for example sponge rubber, which make its use by the patient comfortable.

Advantageously the longitudinal elements 3, 3' can be connected to immobilization straps 8, for example of the spider type. The longitudinal elements 3, 3' can have one or more through ports, not shown in the figures, for the passage of immobilization straps.

The body 1 further comprises connection means 4 to a device for carrying the patient, for example a spinal board 7.

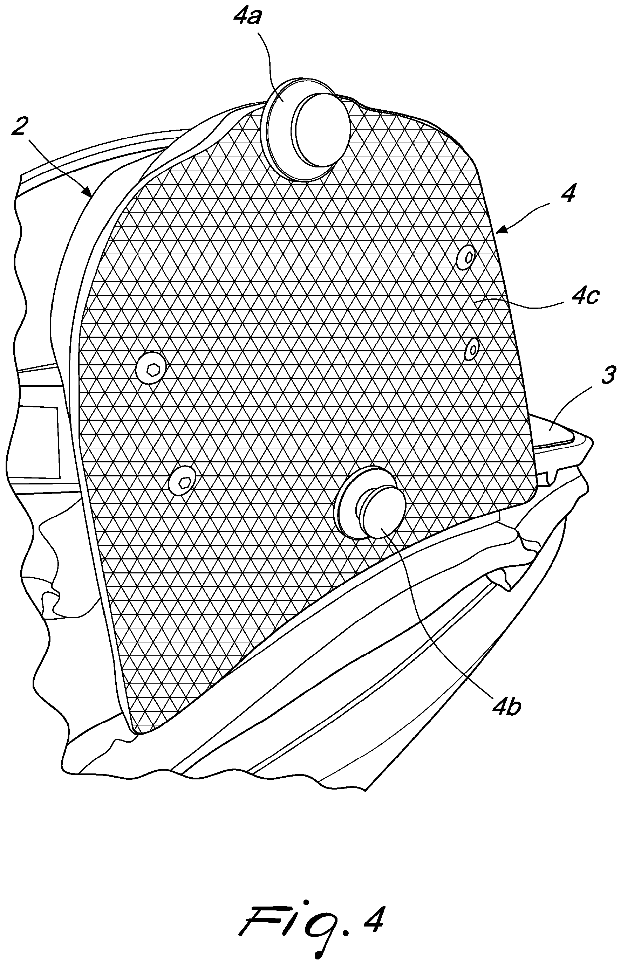

Preferably, such means are arranged on a board 4c that is connected, for example attached, screwed or fixed with equivalent techniques, to the other side of the body 1 on which the patient is laid, as shown in Figure 4.

Preferably, the connection means 4 comprise one or more protrusions 4a, 4b which are connected to the body 1 and are adapted to be inserted slideably into a guide present on a device for carrying the patient. Preferably the protrusions 4a, 4b are provided as discs, preferably made of aluminum and of diameter equal to approximately 1cm, connected to the board by way of rod-like elements which protrude from the board proper. Preferably the protrusions 4a, 4b are aligned and spaced apart for example by a distance equal to approximately 10 cm. Advantageously, the protrusions 4a, 4b make it possible to easily and safely couple the system to a device for carrying the patient, such as a spinal board 7.

The protrusions 4a, 4b are adapted to be inserted slideably into a slot 7c of a device for carrying the patient, in particular of a spinal board 7.

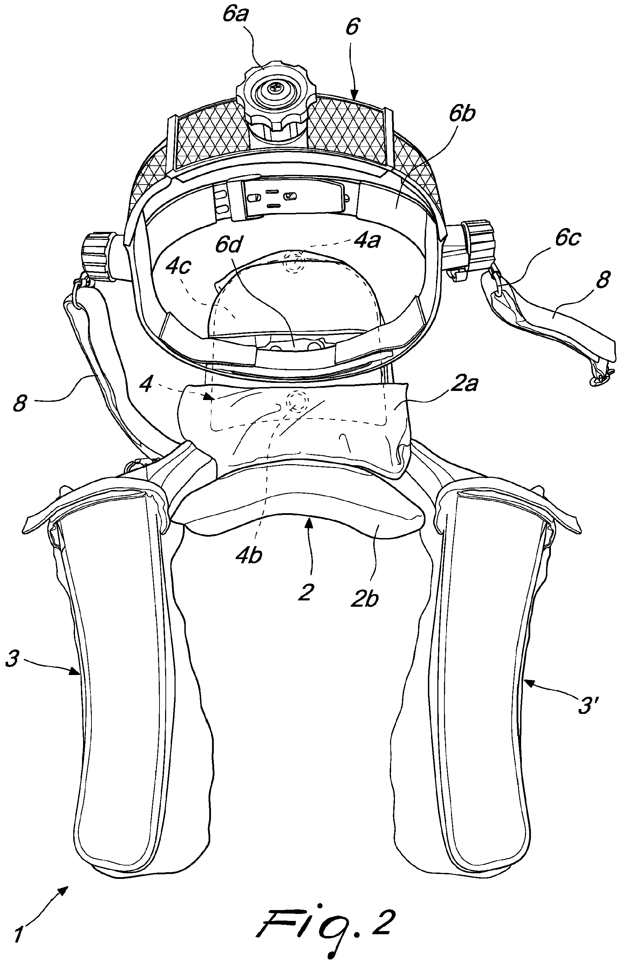

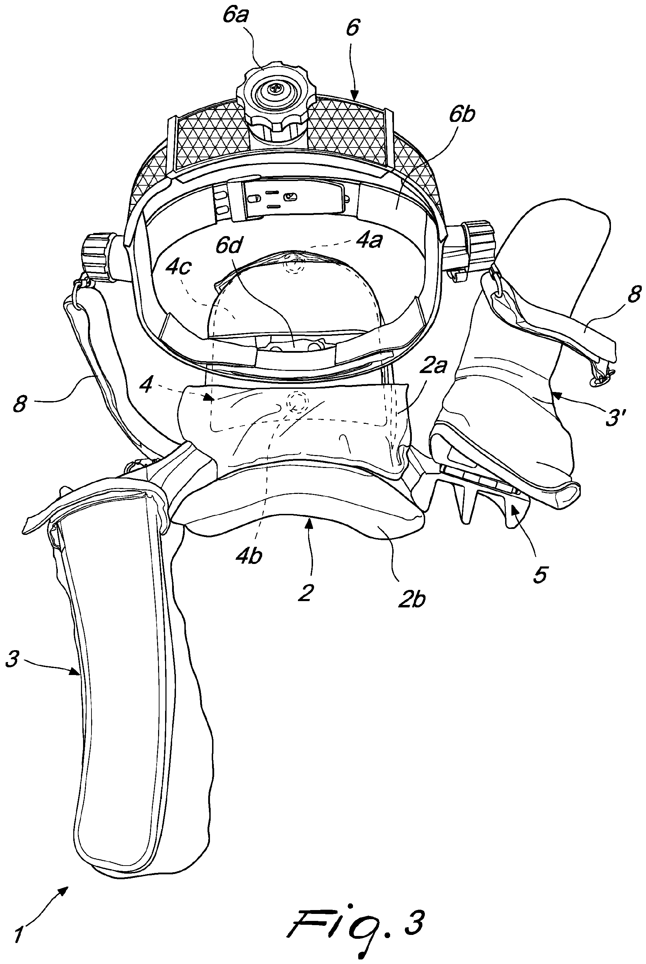

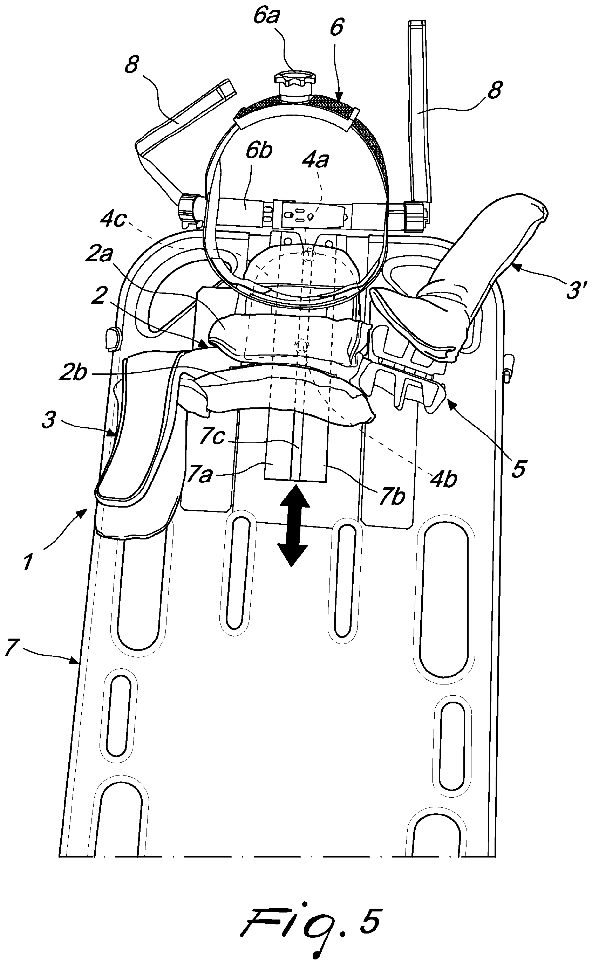

The body 1 further comprises means 5 of rotation (at least partial, for example through 180°) of at least one of these longitudinal elements with respect to the collar, as shown in Figure 5. In this manner the system can pass from the position shown in Figure 2 (or 6), which can be used for carrying the patient, to the position shown in Figure 3 (or 5) and vice versa, which is adapted to putting the system on/removing the system from the patient. For example, the means 5 can be provided as a hinge adapted to pivot the element 3' to the collar 2, for example at the height of the seat 2b adapted to be placed on the shoulders of the patient. By virtue of the rotation of the longitudinal element 3', it is possible to make the operations of applying the system to the patient simpler.

Advantageously, the system further comprises an adjustable front band 6 that is adapted to engage the forehead of the patient, the band comprising an adjustment system 6a, preferably of the type of an adjustment ring, in order to cause the constriction and widening of the band as a function of the dimensions of the head of the patient.

Advantageously the adjustment system 6a is arranged so as to correspond to the frontal region of the head of the patient, and it allows the optimal adjustment of the band so that it better adheres to the cranial circumference of the patient affected by the trauma.

The front band 6 comprises means of connection to the body 1. In a preferred embodiment, such means of connection to the body 1 are provided by way of a hinge that is adapted to adjust the inclination of the front band 6 with respect to the body 1, in this manner the operation to apply the front band 6 to the patient is simpler. Such hinge is preferably arranged in a portion of the collar 2 adjacent to the seat 2a adapted to accommodate the head of the patient.

Preferably the hinge can also be slideably connected to a guide arranged in the body 1 so as to also allow the height of the band to be adjusted.

Preferably, the front band 6 comprises rings 6c for connection to a spring-clip of a strap 8 so as to secure the front band 6, and therefore the system to which it is connected, to a spinal board.

Preferably, on opposite sides of the front band 6 there can be other retention systems composed of two tilting eyelets which are connected by way of two snap-hooks for rapid coupling/decoupling to two adjustable Velcro® straps, the function of which is to safely secure the front band 6 to the spinal board.

Preferably the front band 6 is provided by way of soft crossed plastic strips (similar to those used under hard hats worn by workers in the construction sector) covered with soft rubber for the entire perimetric arc of all the strips, so as to offer the maximum comfort when positioned on the front of the patient.

Advantageously it is also possible to arrange a covering 6a on the front band 6 for the head of the patient, for example a hemispherical cap or an additional band, rigid or semi-rigid, that partially covers the head and immobilizes it. Such covering is connected, for example rigidly to the band 6, so as to offer greater protection to the patient and greater stability to the head.

The system further comprises a spinal board 7 provided with a slot 7c that has an opening for inserting the protrusions 4a, 4b. The protrusions 4a, 4b are insertable into the slot 7c by way of an opening present on the slot 7c, which is preferably provided on an end of the slot proper. The slot 7c defines a guide for sliding the protrusions 4a, 4b along the longitudinal axis of the spinal board 7. The slot 7c is preferably configured to allow the sliding of the protrusions 4a, 4b only along the longitudinal axis of the spinal board 7. However, the slot 7c does not allow movements along a transverse axis: in this manner once the protrusions 4a, 4b are inserted into the slot and locked for example using a retention element, the system is locked and firmly connected to the spinal board 7.

In the preferred embodiment, the slot 7c is defined as the space comprised between two plates 7a, 7b for example made of aluminum, preferably of length equal to approximately 30 cm. Such plates are fixed to the spinal board 7 and spaced apart by a suitable amount (for example 1 cm) so as to create the slot 7c in which the protrusions 4a and 4b can slide.

Advantageously the slot 7c has a width that is such as to not allow the extraction of the protrusions 4a,4b along the axis perpendicular to the board 7. For example, if the protrusions have a diameter of 1 cm, then the slot 7c has a width smaller than 1 cm and a depth, for example 2 cm, adapted to accommodate the protrusions 4a, 4b.

Thus it has been shown that the system described achieves the intended aim and objects. In particular, it has been seen that the system thus conceived makes it possible to overcome the qualitative limitations of the background art by enabling cervical immobilization and facilitating the operations of carrying the patient. The system is composed of a series of elements that, assembled together, protect the cervical column of the patient from traumas owing to maneuvers of the first response personnel.

Such system by virtue of its particular characteristics can be easily accommodated on the trapezius muscles of the shoulders of the patient, allowing an alignment of the cervical column and an adequate and comfortable seating of the head thus ensuring that the patient assumes a suitable position during first response operations and carrying. The presence of the front band 6 and of the spinal board 7, both optional, makes it possible to improve the holding of the correct position of the patient to be carried, and make that position even more stable.

Furthermore by virtue of the hinges, through ports, and snap-hooks, the system can also advantageously be connected to other conventional immobilization systems such as a cervical collar and various straps of the spider type.

Clearly, numerous modifications are evident and can be readily executed by the person skilled in the art without extending beyond the scope of protection of the present invention.

Hence, the scope of protection of the claims shall not be limited by the explanations or by the preferred embodiments illustrated in the description by way of examples, but rather the claims shall comprise all the patentable characteristics of novelty that reside in the present invention, including all the characteristics that would be considered as equivalent by the person skilled in the art.

The disclosures in Italian Patent Application No. 102016000035161 (UA2016A002340) from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A system for immobilizing the head and the cervical region of a patient, which comprises a body (1) that comprises a collar (2) adapted to receive the cervical part and the head of a patient and at least two longitudinal elements (3, 3') which are connected to the collar (2) and are configured to engage respectively a right portion and a left portion of the chest of the patient, **characterized in that** it comprises means (4) of connection to a device for carrying the patient and means (5) of rotation of at least one (3') of said longitudinal elements (3, 3') with respect to said collar (2).

2. The system according to claim 1, **characterized in that** said connection means (4) comprise one or more protrusions (4a, 4b), which are connected to said body (1), preferably by way of a board (7c), said protrusions (4a, 4b) being adapted to be inserted slideably into a guide (7a, 7b, 7c) of a device for carrying the patient.

3. The system according to claim 1 or 2, **characterized in that** said system comprises strap connection means (8) for immobilizing the patient, said straps being preferably of the spider type.

4. The system according to at least one of the preceding claims, **characterized in that** said longitudinal elements (3, 3') have at least one through port for the passage of straps (8) for immobilizing the patient.

5. The system according to at least one of the preceding claims, **characterized in that** said collar (2) comprises a first padding and a second padding, which are arranged respectively in a seat (2a) that is adapted to receive the head of the patient and a seat (2b) that is adapted to receive the cervical part of the patient.

6. The system according to at least one of the preceding claims, **characterized in that** it comprises further an adjustable front band (6) that is adapted to engage the forehead of the patient, said adjustable band comprising an adjustment system (6a), preferably of the type with an adjustment ring, which is adapted to cause the constriction and widening of the front band (6) as a function of the dimensions of the head of the patient; the front band (6) comprising means of connection to said body (1).

7. The system according to claim 6, **characterized in that** said front band (6) comprises rings (6c) for connection to a spring-clip connected to a strap (8) for immobilizing the patient.

8. The system according to at least one of the preceding claims, **characterized in that** it comprises a hinge (6d) for connection to said front band (6), which is accommodated in said body (1), said hinge (6d) being adapted to adjust the inclination of said front band (6) with respect to said body (1).

9. The system according to claim 8, **characterized in that** said hinge (6d) is further configured to slide along a longitudinal axis of said body (1).

10. The system according to at least one of the preceding claims 4 to 9, **characterized in that** it comprises further a covering (6b) for the head of the patient, said covering being connectable/connected to said front band (6).

11. The system according to at least one of the preceding claims 2 to 10, **characterized in that** it comprises a spinal board (7) that is provided with a guide that is configured for the insertion and sliding of said protrusions (4a, 4b).

12. The system according to claim 11, **characterized in that** said guide is constituted by two plates (7a, 7b) which are spaced apart so as to define a slot (7c) adapted to allow the sliding of said protrusions (4a, 4b) along the longitudinal axis of said spinal board (7), said slot (7c) being preferably configured to allow the sliding of said protrusions (4a, 4b) only along the longitudinal axis of said spinal board.

13. An adjustable front band (6), adapted to engage the forehead of a patient, said front band (6) comprising an adjustment system (6a) preferably of the type of an adjustment ring, in order to cause the constriction and widening of the front band (6) as a function of the dimensions of the head of the patient; the front band (6) comprising means of connection to a system according to at least one of the preceding claims.

14. A spinal board (7) provided with a seat that is adapted to accommodate a guide that is adapted to engage connecting means (4a, 4b) of a system according to at least one of the preceding claims 1 to 12.
